# EUROPEAN PATENT SPECIFICATION

(11) **EP 3 074 383 B1**
(45) Date of publication and mention of the grant of the patent: **10.10.2018**
(21) Application number: 14827535.7
(22) Date of filing: 24.11.2014
(51) Int. Cl.: C07D 401/04

(54) **IMPROVED PROCESS FOR THE PREPARATION OF POMALIDOMIDE AND ITS PURIFICATION**
VERBESSERTES VERFAHREN ZUR HERSTELLUNG VON POMALIDOMID UND DESSEN REINIGUNG
PROCÉCÉ DE PRÉPARATION DE POMALIDOMIDE AMÉLIORÉ ET PROCÉDÉ DE PURIFICATION DE POMALIDOMIDE

(30) Priority: 25.11.2013 IN 5409CH2013; 30.01.2014 IN 424CH2014
(43) Date of publication of application: 05.10.2016
(73) Proprietor: Mylan Laboratories Ltd., Hyderabad 500033 (IN)
(72) Inventor: GORE, Vinayak, Hyderabad 500033 (IN); SHUKLA, Vinay Kumar, Hyderabad 500033 (IN); SHINDE, Dhananjay, Hyderabad 500033 (IN); PRAKASH, Bansode, Hyderabad 500033 (IN)
(74) Representative: Cooke, Richard Spencer
(86) International application number: PCT/IB2014/066285
(87) International publication number: WO 2015/075694

(56) References cited:
- WO-A1-2013/126326
- WO-A1-2013/126326
- WO-A1-2013/126326
- WO-A2-2012/177678
- WO-A2-2012/177678
- WO-A2-2014/170909
- WO-A2-2014/170909
- WO-A2-2014/170909
- CN-A- 103 275 062
- CN-A- 103 275 062
- CN-A- 103 275 062
- CN-A- 103 288 797
- CN-A- 103 288 797
- CN-A- 103 288 797
- US-B1- 6 335 349
- US-B1- 6 335 349
- MULLER G W ET AL: "Amino-substituted thalidomide analogs: potent inhibitors of TNF-alpha production", BIOORGANIC & MEDICINAL CHEMISTRY LETTERS, PERGAMON, AMSTERDAM, NL, vol. 9, no. 11, 7 June 1999 (1999-06-07), pages 1625-1630, XP004169632, ISSN: 0960-894X, DOI: 10.1016/S0960-894X(99)00250-4
- MULLER G W ET AL: "Amino-substituted thalidomide analogs: potent inhibitors of TNF-alpha production", BIOORGANIC & MEDICINAL CHEMISTRY LETTERS, PERGAMON, AMSTERDAM, NL, vol. 9, no. 11, 7 June 1999 (1999-06-07), pages 1625-1630, XP004169632, ISSN: 0960-894X, DOI: 10.1016/S0960-894X(99)00250-4

## Description

### CROSS-REFERENCE TO RELATED APPLICATIONS

This application claims priority from Indian patent applications 5409/CHE/2013 filed on Nov. 25, 2013 and 424/CHE/2014 filed on Jan, 30, 2014.

### FIELD OF THE INVENTION:

The present invention relates to an improved process for the preparation of pomalidomide and its purification.

### BACKGROUND OF THE INVENTION:

Pomalidomide is chemically known as (RS)-4-amino-2-(2,6-dioxo-piperidin-3-yl)-isoindoline-1,3-dione and structurally represented as below:

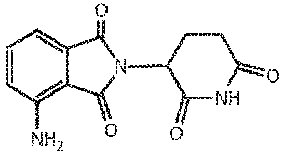

Pomalidomide is an immunomodulatory antineoplastic agent. Pomalidomide is marketed with the brand name POMALYST®. It is indicated for the treatment of relapsed and refractory multiple myeloma.

Several references disclose methods for the preparation and purification of pomalidomide. For example, U.S. Pat. Nos. 5,635,517; 6,335,349; and 7,994,327 disclose processes for the preparation of pomalidomide. Additionally, Chinese Pat. No. 103288797 also discloses a process for the purification of pomalidomide.

The present disclosure provides an improved process for the preparation of pomalidomide with a purity greater than about 99%. The process also results in a high yield, is simple, cost effective, and feasible for large scale production.

### SUMMARY OF THE INVENTION:

The present disclosure provides a process for the preparation of pomalidomide that may include the steps of:
a) reacting nitro phthalic acid with 3-amino-piperidine-2,6-dione or its salt in the presence of a coupling agent and a suitable solvent to obtain 3-(3-nitrophthalimido)-piperidine-2,6-dione, 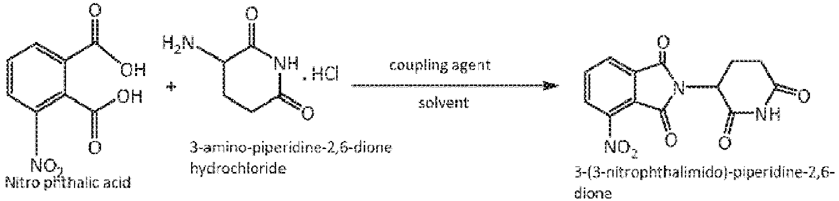
b) reducing 3-(3-nitrophthalimido)-piperidine-2,6-dione in the presence of a solvent and catalyst to obtain pomalidomide.

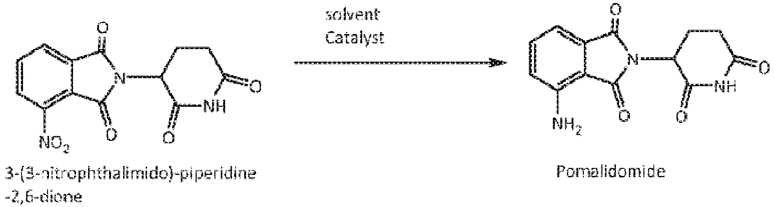

Another aspect of the present disclosure provides a process for the preparation of thalidomide comprising the steps of reacting phthalic acid with 3-amino-piperidine-2,6-dione or its salt to give 2-(2,6-dioxopiperidin-3-yl)-isoindole-1,3-dione, commonly known as thalidomide.

### BRIEF DESCRIPTION OF THE DRAWING:

Further aspects of the present disclosure together with additional features contributing thereto and advantages accruing there from will be apparent from the following description of embodiments of the disclosure which are shown in the accompanying drawing figures wherein:

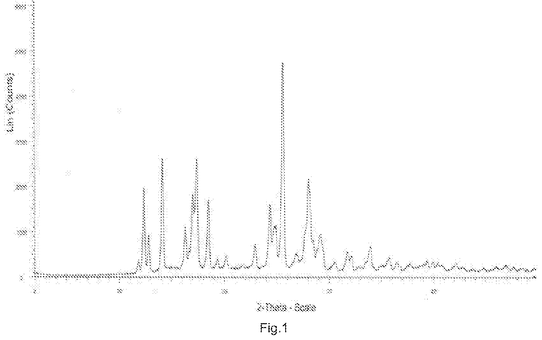

Figure 1 is an X-ray powder diffractogram of pomalidomide prepared and purified according to the present disclosure.

### DETAILED DESCRIPTION OF THE INVENTION:

It is to be understood that the description of the present invention has been simplified to illustrate elements that are relevant for a clear understanding of the invention, while eliminating, for purposes of clarity, other elements that may be well known.

The present invention encompasses novel synthetic schemes for the synthesis of pomalidomide and thalidomide. These schemes provide an improved, efficient method for the synthesis of pomalidomide at a high yield and purity.

More specifically, the present disclosure relates to an improved process for the preparation of pomalidomide.

In one embodiment, the present disclosure provides a process for the preparation of pomalidomide that may include the following steps:
a) reacting nitro-substituted phthalic acid with 3-amino-piperidine-2,6-dione or its salt in the presence of a coupling agent and a solvent to obtain 3-(3-nitrophthalimido)-piperidine-2,6-dione, 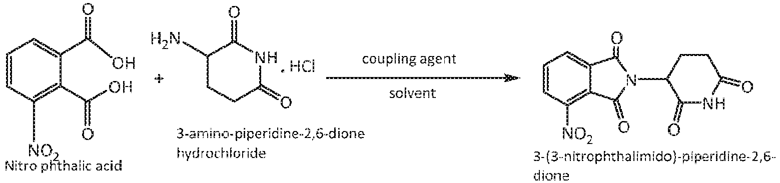
b) reducing 3-(3-nitrophthalimido)-piperidine-2,6-dione in the presence of a solvent and catalyst to obtain pomalidomide.

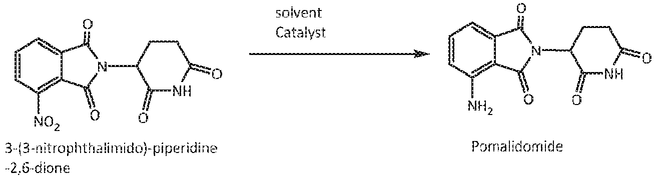

According to the present disclosure, nitrophthalic acid with 3-amino-piperidine-2,6-dione or its salt is converted to 3-(3-nitrophthalimido)-piperidine-2,6-dione in the presence of a coupling agent and a solvent per step (a) above. The reaction may be performed at about 25°C to about 80°C for about 5 to 18 hours. Within the context of the present disclosure, the coupling agent may include, as examples, 1,1-carbonyldiimadazole, dicyclohexylcarbodiimide, diisopropylcarbodiimide, 2-chloro-4,6-dimethoxy-1,3,5-triazine (CDMT), dimethylaminopyridine, and mixtures thereof. One of skill in the art will readily recognize additional compounds that may be useful in activating the carboxylic acid groups of nitrophthalic acid so that 3-amino-piperidine-2,6-dione hydrochloride may react and create the pyrrolidine ring to couple together nitrophthalic acid and the 3-amino-piperidine-2,6-dione. The solvent may include, as examples, nitriles (such as acetonitrile and propionitrile), acid amides (such as N,N-dimethylformamide and dimethylacetamide), and cyclic ethers (such as tetrohydrofuran and 1,4-dioxane). Again, one of skill in the art will recognize other solvents that may be suitable for use in this reaction.

According to the present disclosure, 3-(3-nitrophthalimido)-piperidine-2,6-dione is then reacted at ambient temperature for about 5-6 hours in the presence of a solvent and catalyst to obtain pomalidomide, per step (b) above. Within the context of the present disclosure, the catalyst may be, for example, palladium on carbon, Raney nickel or reducing agents such as iron-hydrochloric acid, zinc-acetic acid, zinc ammonium chloride, bubbled hydrogen (per Example 3 below), and sodium dithionite. One skilled in the art may recognize a variety of other compounds that may be useful reducing agents for use in this step of the reaction disclosed herein. The solvent used in this particular step of the process may be, for example, an acid amide (such as, N,N-dimethylformamide and N,N-dimethyl acetamide), dimethyl sulfoxide, a nitrite (such as acetonitrile or propionitrile), or aliphatic alcohols (such as methanol, isopropanol and mixtures thereof). Again, one skilled in the art may recognize a variety of other solvents that may be useful in performing this reaction.

Another aspect of the present disclosure provides a process for the preparation of thalidomide, wherein phthalic acid is reacted with 3-amino-piperidine-2,6-dione or its salt in the presence of a coupling agent and solvent to give 2-(2,6-dioxopiperidin-3-yl)-isoindole-1,3-dione (thalidomide). Within the context of this disclosure, the coupling agents may include, as examples, 1,1-carbonyidiimidazole, dicyclohexylcarbodiimide, diisopropylcarbodiimide, 2-chloro-4,6-dimethoxy-1,3,5-triazine (CDMT), dimethylaminopyridine, and mixtures thereof. One of skill in the art will readily recognize additional compounds that may be useful in activating the carboxylic acid groups of nitrophthalic acid so that 3-amino-piperidine-2,6-dione hydrochloride may react and create the pyrrolidine ring to couple together phthalic acid and the 3-amino-piperidine-2,6-dione. The solvent may include, as examples, nitrites (such as acetonitrile and propionitrile), acid amides (such as N,N-dimethylformamide and dimethyl acetamide), and cyclic ethers (such as tetrahydrofuran and 1,4-dioxane). Again, one of skill in the art will recognize other solvents that may be suitable for use in this reaction.

Another aspect of the present disclosure is to provide a process for the purification of pomalidomide which may include the following steps:
a) dissolving pomalidomide in an organic solvent or mixture of solvents,
b) adding an anti-solvent, and
c) isolating substantially pure pomalidomide.

According to the present disclosure, pomalidomide is dissolved in an organic solvent. Within the context of the current invention, the organic solvent used in step (a) is dimethyl sulfoxide, diethyl sulfoxide, di-n-propyl sulfoxide, di-or tetra-n-butyl sulfone sulfoxide, acetone, methyl isobutyl ketone, or mixtures thereof.

According to the present disclosure, anti-solvent is then added to the pomalidomide/organic solvent solution. Within the context of the present specification, an anti-solvent is a fluid in which the product is insoluble, thus permitting more facile isolation of the product. For the present invention, anti-solvents are alcohols, ethers, water, or mixtures thereof. Suitable alcohols include methanol, ethanol, n-propanol, isopropanol, and n-butanol. Suitable ethers include diethyl ether, tert-butyl methyl ether, and diisopropyl ether.

Within the context of the current invention, the final pomalidomide product prepared by the processes described herein may yield a product with a purity greater than about 99.7% as measured by HPLC. Individual identified chemical impurities in the final pomalidomide product may be present at quantities less than about 0.10%.

A crystalline form of pomalidomide, prepared and purified according to the processes disclosed in the present invention, may be characterized by powder X-ray diffraction (PXRD), and have a PXRD pattern as shown in Fig. 1 and having peaks at 12.1, 13.9, 17.1, 18.3, 24.2, 25.4, 27.9 ± 0.2 2Θ.

The pomalidomide as synthesized and purified by the methods disclosed herein may be useful in generating pharmaceutical dosage forms suitable for administration to patients in need thereof. The dosage form may be an oral dosage form and in some embodiments, the oral dosage form may be a capsule. The capsule may include appropriate excipients including mannitol, pre-gelatinized starch, and sodium stearyl fumarate. Such formulations may be useful in the treatment of multiple myeloma. Formulations of pomalidomide are particularly useful for patients having multiple myeloma who have received at least two prior therapies including lenalidomide and bortezomib and have demonstrated disease progression on or within sixty days of completion of the last therapy.

In view of the above description and the examples below, one of ordinary skill in the art will be able to practice the invention as claimed without undue experimentation. The foregoing will be better understood with reference to the following examples that detail certain procedures for the preparation of molecules, compositions and formulations according to the present invention. All references made to these examples are for the purposes of illustration. The following examples should not be considered exhaustive, but merely illustrative of only a few of the many aspects and embodiments contemplated by the present disclosure.

### Examples:

### Example 1: Synthesis of 1,3-dioxo-2-(2,6-dioxopiperidin-3-yl)-4-nitro-isoindoline-dione (3-nitrophthalidomide)

To the stirred mixture of 3-nitrophthalic acid (25.0 g or 0.12 mole) in acetonitrile (175 ml) was added 1,1-carbonyldiimidazole (CDI) (42.3 g or 0.26 mole) under nitrogen atmosphere at ambient temperature. To this mixture, 3-aminopiperidine-2,6-dione hydrochloride (19.5g or 0.12 mole) was added, and the reaction mixture was heated to 75 to 80 °C until the reaction was completed as monitored by TLC. After completion of the reaction, the solvent was distilled out under reduced pressure. Water (375 ml) was added to the reaction mass, and the reaction mass was slowly cooled at 0 to 5 °C while stirring. The isolated solid was filtered, washed with water, then by methanol, and suck dried. Finally the isolated solid was dried at 55 to 60 °C under vacuum until constant weight to obtain 3-nitrophthalidomide. Yield: 28.2 g, 78.5% (molar) (HPLC purity ∼99.5%).

### Example 2: Synthesis of 2-(2,6-dioxopiperidin-3-yl)-isoindole-1-dione (Thalidomide)

1,1-carbonyldiimidazole (21.5 g or 0.13 mole) was added to a stirred mixture of phthalic acid (10.0 g or 0.06 mole) in acetonitrile (100 ml) maintained under nitrogen at ambient temperature. To this mixture, 3-aminopiperidine-2,6-dione hydrochloride (9.9 g or 0.06 mole) was added, and the reaction mixture was stirred at 25 to 30 °C until the reaction was completed as monitored by TLC. After completion of the reaction, the solvent was distilled out under reduced pressure. Water (100 ml) was added to the reaction mass and the reaction mass was slowly cooled at 0 to 5 °C while stirring. The isolated solid was filtered, washed with water, then by methanol, and suck dried. Finally the isolated sold was dried at 55 to 60 °C under vacuum until constant weight to get thalidomide. Yield: 11.6 g, 75.2% (molar). Purity by HPLC 99.13%

### Example 3: Synthesis of 4-amino-2-(2,6-dioxopiperidin-3-yl)-isoindoline-1,3-dione (Pomalidomide)

Catalyst palladium (10%) on carbon (0.5 g. or 5% w/w of substrate) was added to a stirred solution of 3-nitrophthalidomide (10 g or 0.033 mole) (Example 1) in N,N-dimethyl formamide (100 ml) maintained under nitrogen. Hydrogen gas was bubbled through the reaction mixture at atmospheric pressure. After completion of hydrogenation reaction, as monitored by TLC, hydrogen bubbling was stopped and the catalyst was separated by filtration of the reaction mixture on celite bed. The filtrate was distilled at 60 to 65 °C under reduced pressure until half of the solvent was removed. The solution was then cooled at ambient temperature and methanol (50 ml) was added while stirring. The solid obtained was filtered, washed with methanol (50 ml), and suck dried to obtain pomalidomide 8.2 g (90% molar yield) having HPLC purity > 99.0%.

### Example 4: Pomalidomide Purification Process

In a reaction vessel, crude (RS) 4-amino-2-(2,6-dioxopiperidine-3-yl)isoindole-1,3-dione (pomalidomide, 10g) was dissolved in dimethylsulfoxide (40 ml) by heating at 60 to 65 °C. The solution was cooled to 25 to 30 °C. To this solution, acetone (40 ml) was added while stirring. After completion of acetone addition, the solution was stirred for 30 minutes and then methanol (40 ml) was added at slow rate while stirring at 25 to 30 °C. The resulting mixture was allowed to stir for one to two hours. The isolated solid was filtered, washed with a methanol-acetone mixture (1:1 v/v, 10 ml) and suck dried. Finally, the material was dried by vacuum at 55 to 60 °C to get pure pomalidomide (8 g, 80% yield) with HPLC purity greater than 99.7%). All known individual chemical impurities were present at quantities less than 0.10 %).

## Claims

1. A process for the synthesis of pomalidomide, comprising the steps of:
a) reacting nitrophthalic acid with 3-amino-piperidine-2,6-dione or its salt in the presence of a coupling agent and a first solvent to obtain 3-(3-nitrophthalimido)-piperidine-2,6-dione; and
b) reducing the 3-(3-nitrophthalimido)-piperidine-2,6-dione in the presence of a second solvent and a catalyst to obtain pomalidomide.

2. The process according to claim 1, wherein the coupling agent is selected from the group consisting of 1,1-carbonyldiimidazole, dicyclohexyl carbodiimide, diisopropyl carbodiimide, 2-chloro-4,6-dimethoxy-1,3,5-triazine(CDMT), and dimethyl aminopyridine.

3. The process of claim 1, wherein the first solvent is selected from the group consisting of acetonitrile, propionitrile, N,N-dimethylformamide, dimethyl acetamide, tetrahydrofuran, 1,4-dioxane, and mixtures thereof.

4. The process according to claim 1, wherein the catalyst is selected from the group consisting of palladium on carbon, Raney nickel, and a reducing agent.

5. The process of claim 4, wherein the reducing agent is selected from the group consisting of iron-hydrochloric acid, zinc-acetic acid, zinc ammonium chloride, bubbled hydrogen, and sodium dithionite.

6. The process of claim 1, wherein the second solvent is selected form the group consisting of N,N-dimethylformamide N,N-dimethyl acetamide, dimethyl sulfoxide, acetonitrile, propionitrile, methanol, isopropanol, and mixtures thereof.

7. The process according to claim 1, wherein the pomalidomide has a purity of greater than about 99.7%.

8. The process according to claim 1, further comprising the steps of:
a) dissolving pomalidomide in an organic solvent,
b) adding an anti-solvent, and
c) isolating pomalidomide with a purity greater than about 99%,
wherein the organic solvent is selected from the group consisting of dimethyl sulfoxide, diethyl sulfoxide, di-n-propyl sulfoxide, di-or tetra-n-butyl sulfone sulfoxide, acetone, methyl isobutyl ketone, and mixtures thereof, and
wherein the anti-solvent is selected from the group consisting of alcohol, ether, water, and mixtures thereof.

9. The process according to claim 8, wherein said alcohol is selected from the group consisting of methanol, ethanol, n-propanol, isopropanol, and n-butanol.

10. The process according to claim 8, wherein said ether is selected from the group consisting of diethyl ether, tert-butyl methyl ether, and diisopropyl ether.

11. The process according to claim 8, wherein said isolating step comprises filtering, drying, and evaporation.

## Patentansprüche

1. Verfahren zur Synthese von Pomalidomid, umfassend die Schritte des:
a) Umsetzens von Nitrophthalsäure mit 3-Aminopiperidin-2,6-dion oder dessen Salz in Gegenwart eines Kopplungsmittels und eines ersten Lösungsmittels, wodurch 3-(3-Nitrophthalimido)piperidin-2,6-dion erhalten wird; und des
b) Reduzierens des 3-(3-Nitrophthalimido)piperidin-2,6-dions in Gegenwart eines zweiten Lösungsmittels und eines Katalysators, wodurch Pomalidomid erhalten wird.

2. Verfahren nach Anspruch 1, wobei das Kopplungsmittel ausgewählt ist aus der Gruppe bestehend aus 1,1-Carbonyldiimidazol, Dicyclohexylcarbodiimid, Diisopropylcarbodiimid, 2-Chlor-4,6-dimethoxy-1,3,5-triazin (CDMT) und Dimethylaminopyridin.

3. Verfahren nach Anspruch 1, wobei das erste Lösungsmittel ausgewählt ist aus der Gruppe bestehend aus Acetonitril, Propionitril, N,N-Dimethylformamid, Dimethylacetamid, Tetrahydrofuran, 1,4-Dioxan und Mischungen davon.

4. Verfahren nach Anspruch 1, wobei der Katalysator ausgewählt ist aus der Gruppe bestehend aus Palladium auf Kohlenstoff, Raney-Nickel und einem Reduktionsmittel.

5. Verfahren nach Anspruch 4, wobei das Reduktionsmittel ausgewählt ist aus der Gruppe bestehend aus Eisen-Salzsäure, Zink-Essigsäure, Zink-Ammoniumchlorid, perlendem Wasserstoff und Natriumdithionit.

6. Verfahren nach Anspruch 1, wobei das zweite Lösungsmittel ausgewählt ist aus der Gruppe bestehend aus N,N-Dimethylformamid, N,N-Dimethylacetamid, Dimethylsulfoxid, Acetonitril, Propionitril, Methanol, Isopropanol und Mischungen davon.

7. Verfahren nach Anspruch 1, wobei das Pomalidomid eine Reinheit von mehr als etwa 99.7 % aufweist.

8. Verfahren nach Anspruch 1, weiterhin umfassend die Schritte des:
a) Auflösens von Pomalidomid in einem organischen Lösungsmittel,
b) Zugebens eines Antilösungsmittels und des
c) Isolierens von Pomalidomid mit einer Reinheit von mehr als etwa 99 %,
wobei das organische Lösungsmittel ausgewählt ist aus der Gruppe bestehend aus Dimethylsulfoxid, Diethylsulfoxid, Di-n-propylsulfoxid, Di- oder Tetra-n-butylsulfonsulfoxid, Aceton, Methylisobutylketon und Mischungen davon und
wobei das Antilösungsmittel ausgewählt ist aus der Gruppe bestehend aus Alkohol, Ether, Wasser und Mischungen davon.

9. Verfahren nach Anspruch 8, wobei der Alkohol ausgewählt ist aus der Gruppe bestehend aus Methanol, Ethanol, n-Propanol, Isopropanol und n-Butanol.

10. Verfahren nach Anspruch 8, wobei der Ether ausgewählt ist aus der Gruppe bestehend aus Diethylether, tert-Butylmethylether und Diisopropylether.

11. Verfahren nach Anspruch 8, wobei der Isolierschritt ein Filtern, Trocknen und Verdampfen umfasst.

## Revendications

1. Procédé de synthèse de pomalidomide, comprenant les étapes :
a) de réaction de l'acide nitrophtalique avec de la 3-amino-pipéridine-2,6-dione ou de son sel en présence d'un agent de couplage et d'un premier solvant pour obtenir de la 3-(3-nitrophthalimido)- pipéridine-2,6-dione ; et
b) de réduction de la 3-(3-nitrophthalimido)-pipéridine-2,6-dione en présence d'un second solvant et d'un catalyseur pour obtenir le pomalidomide.

2. Procédé selon la revendication 1, dans lequel l'agent de couplage est choisi dans le groupe constitué du 1,1-carbonyldiimidazole, du dicyclohexyl carbodiimide, du diisopropyl carbodiimide, de la 2-chloro-4,6-diméthoxy-1,3,5-triazine(CDMT), et de la diméthyl aminopyridine.

3. Procédé selon la revendication 1, dans lequel le premier solvant est choisi dans le groupe constitué de l'acétonitrile, du propionitrile, du N,N-diméthylformamide, du diméthyl acétamide, du tétrahydrofurane, du 1,4-dioxane, et de leurs mélanges.

4. Procédé selon la revendication 1, dans lequel le catalyseur est choisi dans le groupe constitué du palladium sur du carbone, du nickel Raney, et d'un agent réducteur.

5. Procédé selon la revendication 4, dans lequel l'agent réducteur est choisi dans le groupe constitué des mélanges fer-acide chlorhydrique, zinc-acide acétique, zinc-chlorure d'ammonium, de bulles d'hydrogène et de dithionite de sodium.

6. Procédé selon la revendication 1, dans lequel le second solvant est choisi dans le groupe constitué du N,N-diméthylformamide, du N,N-diméthyl acétamide, du diméthyl sulfoxyde, de l'acétonitrile, du propionitrile, du méthanol, de l'isopropanol, et de mélanges de ceux-ci.

7. Procédé selon la revendication 1, dans lequel le pomalidomide a une pureté supérieure à environ 99,7 %.

8. Procédé selon la revendication 1, comprenant en outre les étapes :
a) de dissolution du pomalidomide dans un solvant organique,
b) d'addition d'un anti-solvant, et
c) d'isolation du pomalidomide avec une pureté supérieure à environ 99 %,
où le solvant organique est choisi dans le groupe constitué du diméthyl sulfoxyde, du diéthyl sulfoxyde, du di-n-propyl sulfoxyde, du di- ou tétra-n-butyl sulfone sulfoxyde, de l'acétone, de la méthyl isobutyl cétone, et de leurs mélanges et
où l'anti-solvant est choisi dans le groupe constitué d'un alcool, d'un éther, de l'eau, et de leurs mélanges.

9. Procédé selon la revendication 8, dans lequel ledit alcool est choisi dans le groupe constitué du méthanol, de l'éthanol, du n-propanol, de l'isopropanol, et du n-butanol.

10. Procédé selon la revendication 8, dans lequel ledit éther est choisi dans le groupe constitué du diéthyl éther, du tert-butyl méthyl éther, et du diisopropyl éther.

11. Procédé selon la revendication 8, dans lequel ladite étape d'isolation comprend une filtration, un séchage, et une évaporation.
